# EUROPÄISCHE PATENTSCHRIFT

(11) **EP 3 287 785 B1**
(45) Veröffentlichungstag und Bekanntmachung des Hinweises auf die Patenterteilung: **07.11.2018**
(21) Anmeldenummer: 16185557.2
(22) Anmeldetag: 24.08.2016
(51) Int. Cl.: G01N 33/558, G01N 33/487

(54) **VORRICHTUNG ZUM VISUELLEN NACHWEIS VON ANALYTEN IN EINER FLÜSSIGEN PROBE**
DEVICE FOR VISUAL DETECTION OF ANALYTES IN A LIQUID SAMPLE
DISPOSITIF DE PREUVE VISUELLE D'ANALYTES DANS UN ÉCHANTILLON LIQUIDE

(43) Veröffentlichungstag der Anmeldung: 28.02.2018
(60) Teilanmeldung: 17194441.6
(73) Patentinhaber: Protzek Gesellschaft für Biomedizinische Technik GmbH, 4133 Prattein (CH)
(72) Erfinder: Protzek, Christoph, 79539 Lörrach (DE)
(74) Vertreter: Patentanwälte Dörner & Kötter PartG mbB

(56) Entgegenhaltungen:
- WO-A1-00/05579
- WO-A1-2014/137858
- WO-A1-2016/195236
- WO-A2-2005/006959
- DE-A1- 19 843 094
- DE-T2- 69 912 904
- DE-U1-202014 002 369
- DE-U1-202016 100 261
- US-A1- 2014 273 269

## Beschreibung

Die Erfindung betrifft eine Vorrichtung zum visuellen Nachweis von Analyten in einer vom menschlichen oder tierischen Organismus stammenden Analysen- oder Flüssigkeitsprobe nach dem Oberbegriff des Patentanspruchs 1.

Vorrichtungen zum Nachweis von Analyten in einer flüssigen Probe, zum Beispiel von Drogen, Medikamenten oder Antikörpern als Zeichen für bestimmte Erkrankungen in einer Körperflüssigkeit oder Feststellungen im Sinne von Konzentrationsangaben sind in unterschiedlichen Ausführungen bekannt. Besonderes Interesse besteht an solchen Geräten, die auch außerhalb eines Labors eingesetzt werden können. Diese müssen tragbar und leicht zu bedienen sein sowie in kurzer Zeit ein zuverlässiges Ergebnis liefern. Insbesondere zur Durchführung von Tests auf Drogenkonsum oder Medikamentenmissbrauch oder dergleichen durch die Polizei ist eine Vorrichtung erforderlich, die auch im Freien bei unterschiedlichen wechselnden Lichtverhältnissen, Wetterverhältnissen und Temperaturen einen derartigen Test ermöglicht. In der DE 20 2014 002 369 U1 wird hierzu ein Gerät vorgeschlagen, in das eine Testkassette einführbar ist und dass mit einer Lichtquelle versehen ist, mittels derer die untere Wand der aus einem lichtdurchlässigem Kunststoff bestehenden Testkassette sowie den Testbereich des in der Testkassette angeordneten Teststreifens durchstrahlt und das Testergebnis ausleuchtet. Die Testkassette ist zur Durchführung eines lateral flow immuno-assays, Aptamer basierter Assay oder Encym immuno assay (EIA) ausgelegt und besteht aus einem unteren Deckel und einem oberen Deckel. Eingeschlossen von diesen beiden Deckeln enthält die Testkassette einen Teststreifen, der innerhalb einer Aufnahme in Position gehalten wird. In dem oberen Deckel befindet sich über dem Probenaufnehmer des Teststreifens eine Einfüllöffnung, durch die eine zu untersuchende flüssige Probe eingegeben werden kann. Über den mittleren Testbereich des Teststreifens befindet sich ein Kontrollfenster, durch welches das Testergebnis abgelesen werden kann. Derartige Kassetten haben sich im mobilen Einsatz bewährt. Nachteilig an den vorbekannten Kassetten ist jedoch, dass dessen Herstellung aufwendig ist und diese relativ viel Platz benötigen.

Hier will die Erfindung Abhilfe schaffen.

DE19843094 A1 enthüllt Testvorrichtungen, bei denen poröse Matrixmaterialien auf inerten Trägern aufgebracht sind. Die inerten Träger können aus beschichteter Pappe hergestellt werden.

Der Erfindung liegt die Aufgabe zu Grunde, eine Vorrichtung zum visuellen und messtechnischen Nachweis von Analyten in einer flüssigen Probe bereitzustellen die einfach aufgebaut ist, für den mobilen Einsatz geeignet ist und die nur wenig Platz beansprucht. Gemäß der Erfindung wird diese Aufgabe durch eine Vorrichtung mit den Merkmalen des Patentanspruchs 1 gelöst.
Mit der Erfindung ist eine Vorrichtung zum visuellen und messtechnischen Nachweis von Analyten in einer flüssigen Probe bereitgestellt die einfach aufgebaut ist, mobil einsetzbar ist und die nur wenig Platz beansprucht. Durch die Ausgestaltung der Vorrichtung durch eine Trägerplatte, die mit einer Teststreifenaufnahme versehen ist, in der ein mit einem Probenaufnahmeabschnitt und einem Testabschnitt versehener Teststreifen angeordnet ist, welche wenigstens eine Teststreifenaufnahme zumindest bereichsweise über eine nicht lösbar mit der Trägerplatte verbundene Deckschicht verschlossen ist, ist eine sehr platzsparende, komprimierte Bauweise ermöglicht. Bevorzugt weist die Deckschicht ein zu dem Kontrollfenster beabstandet angeordnetes Probeaufgabefenster auf, dass eine Probeaufgabe auf den Probenaufnahmeabschnitt wenigstens eines Teststreifens ermöglicht.

Dadurch, dass die Trägerplatte aus Pappe hergestellt ist, wobei vorhandene Teststreifenaufnahmen und/oder Reservoire durch Einprägungen ausgebildet sind, ist eine umweltschonende Entsorgung der Vorrichtung erzielt. Hierzu ist vorzugsweise auch der Deckel aus Pappe ausgebildet. Alternativ können vorhandene Teststreifenaufnahmen und/oder Reservoire auch durch Ausnehmungen einer zwischen Deckfolie und Trägerplatte angeordneten Zwischenschicht ausgebildet sein.
In Weiterbildung der Erfindung sind wenigstens zwei parallel zueinander angeordnete, jeweils einen Teststreifen aufnehmende Teststreifenaufnahmen angeordnet, die an einem Ende in einem gemeinsamen Reservoir münden, in dem die Probeaufnahmeabschnitte der Teststreifen angeordnet sind und über dem das Probeaufgabefenster der Deckschicht positioniert ist. Hierdurch ist eine gleichzeitige Probeaufgabe auf alle in dem Reservoir mündenden Probeaufnahmeabschnitten der Teststreifen ermöglicht. Über die Ausgestaltung des Reservoirs ist zudem eine ausreichende Zufuhr an Flüssigkeitsprobe gewährleistet, sowie auch eine homogene Verteilung der Flüssigkeit auf dem vorzugsweise mit einer Separationsfolie (Probenvlies) versehenen Probeaufnahmeabschnitt der Teststreifen.

In Ausgestaltung der Erfindung ist im Bereich des Probeaufgabefensters zwischen Deckschicht und Teststreifen ein Probeaufnahmevlies angeordnet. Hierdurch ist eine gleichmäßige Beaufschlagung der Teststreifen mit der Flüssigkeitsprobe bewirkt.

In Weiterbildung der Erfindung ist die Deckschicht aus einer Folie gebildet, die bevorzugt im Wesentlichen flächig mit der Trägerplatte und den von der Trägerplatte aufgenommenen Teststreifen verklebt ist. Hierdurch ist ein Schutz der Teststreifen vor Kontamination erzielt. Dabei ist die Folie vorteilhaft aus verrottbarem Material hergestellt. Dass wenigstens eine Kontrollfenster ist vorteilhaft durch jeweils einen transparenten Bereich der Folie gebildet.

In weiterer Ausgestaltung der Erfindung sind zwei schwenkbar miteinander verbundene Trägerplatten angeordnet, die jeweils auf einer Hälfte ihrer Oberfläche mit Teststreifenaufnahmen versehen sind, derart, dass im eingeklapptem Zustand jeweils die mit Teststreifenaufnahmen versehene Hälfte einer Trägerplatte an der keine Teststreifenaufnahmen aufweisenden Hälfte der an dieser anliegenden anderen Trägerplatte anliegt. Hierdurch ist eine Anordnung von Teststreifen auf beiden Seiten der Vorrichtung erzielt, wodurch die mögliche Anzahl an Teststreifen erhöht ist. Dabei ist zugleich eine dünne Bauweise erzielt.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:
- Figur 1: die schematische Darstellung einer Vorrichtung zum visuellen Nachweis von Analyten in einer flüssigen Probe in Explosionsdarstellung und
- Figur 2: die schematische Darstellung eines Teststreifen der Vorrichtung aus Figur 1.

Die als Ausführungsbeispiel gewählte Vorrichtung zum visuellen Nachweis von Analyten in einer flüssigen Probe besteht im Wesentlichen aus zwei entlang ihrer Längsseite schwenkbar miteinander verbundenen Trägerplatten 1, auf denen jeweils eine Zwischenschicht 2 aufgebracht ist, die Teststreifen 3 aufnimmt und die mit einer Deckschicht 4 versehen ist.

Die Trägerplatten 1 sind im Ausführungsbeispiel aus Pappe hergestellt und aus einem einzigen mittig mit einer Knickfalz 11 versehenen Kartonzuschnitt gebildet.

Die Zwischenlage 2 ist im Ausführungsbeispiel aus Wellpappe hergestellt und mit einer transparenten, flüssigkeitsdichtenden Folie beschichtet, um ein Eindringen von Probenflüssigkeit in den Karton zu verhindern. Alternativ kann die Zwischenschicht 2 auch mit einem wasserdichten, vorzugsweise umweltgerechten Lack versehen sein. In der Zwischenschicht 2 ist eine Ausnehmung 21 eingebracht, durch die jeweils zwei Teststreifenaufnahmen 12 gebildet sind, die in einem Reservoir 13 münden. Die Teststreifenaufnahmen 12 nehmen Teststreifen 3 auf.

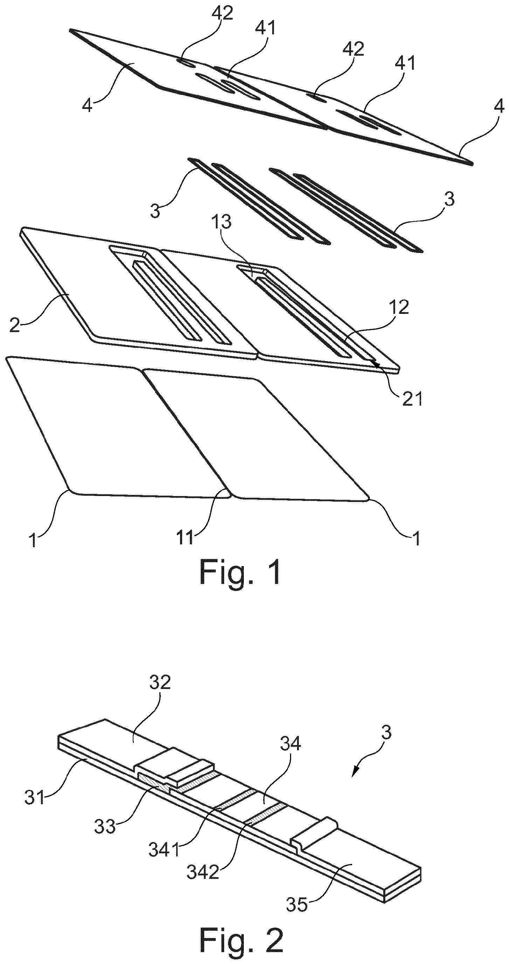

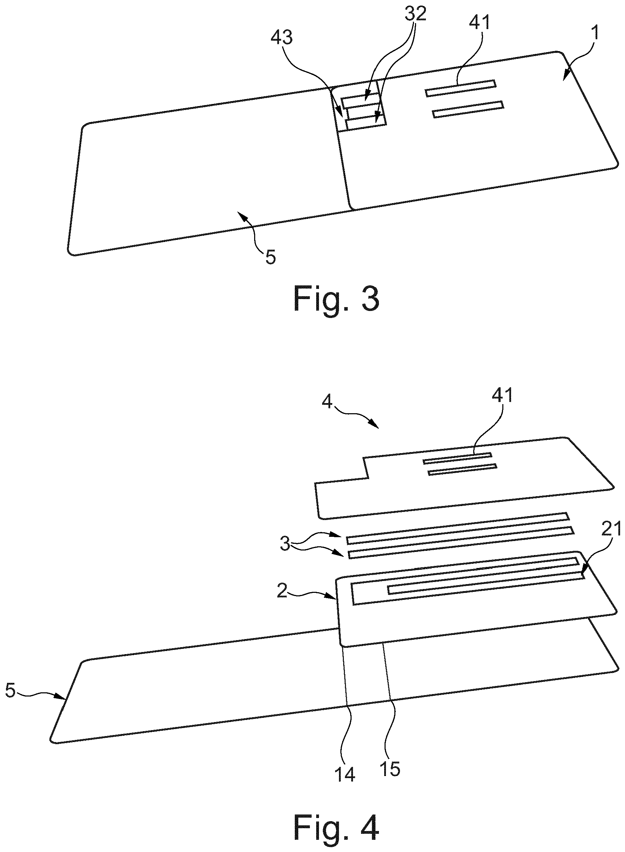

Der Teststreifen 3 ist in Figur 2 im Stufenschnitt dargestellt. Der Teststreifen 3 weist eine Trägerfolie 31 auf, auf der an einem Ende ein Probeaufnahmeabschnitt 32 angeordnet ist. Unmittelbar benachbart und teilweise unter dem Probeaufnahmeabschnitt 32 befindet sich auf der Trägerfolie 31 ein Antikörperbelag 33, der Antikörper enthält, die mit Goldpartikeln konjugiert sind und die zu einem festzustellenden Analyten bzw. zu einem Antigen dieses Analyten passen und in eine immunologische Reaktion treten können. An den Antikörperbelagabschnitt 33 schließt sich ein Testabschnitt 34 an, der in Form einer Membran ausgebildet ist, auf den sich beabstandet zueinander eine Testlinie 341 und eine Kontrolllinie 342 befinden, die durch in einem Reagenz für den Nachweis eines bestimmten Analyten enthaltenen Antigen ausgebildet sind und vor Aufgabe einer Probe unsichtbar sind. Der Probenaufnahmeabschnitt 32 des Teststreifens 3 befindet sich unterhalb der Probenaufgabefenster 42 und der Testabschnitt 34 befindet sich unterhalb des Kontrollfensters 41 der auf der Zwischenschicht 2 aufgebrachten Deckschicht 4.
Bei der Durchführung eines lateral flow assays zeigt die Testlinie 341 das Ergebnis des Tests an; die Kontrolllinie 342 gibt an, ob eine ausreichende Probemenge aufgegeben worden ist, also ob der Test valide ist und seine Durchführung sachgemäß erfolgte. An seinem dem Probenaufnahmeabschnitt 32 gegenüberliegenden Ende ist an den Teststreifen 3 ein Absorberabschnitt 35 angeordnet.

Die Deckschicht 4 ist im Ausführungsbeispiel aus einer verrottbaren, farbigen Folie gebildet, die mit transparent ausgebildeten, klaren, reflektionsarmen Kontrollfenstern 41 versehen ist. Beabstandet zu den Kontrollfenstern 41 ist ein Probeaufgabefenster 42 angeordnet, dass durch eine in der Deckschicht 4 eingebrachte Ausnehmung gebildet ist. Das Probeaufgabefenster 42 ist derart angeordnet, dass es über den Probeaufnahmeabschnitten 32 beider Teststreifen 3 positioniert ist. Im Ausführungsbeispiel ist zwischen den Teststreifen 3 und dem Probeaufgabefenster 42 der Deckschicht 4 ein - nicht dargestelltes - Probeaufnahmevlies angeordnet, dass die Probenaufnahmeabschnitte 32 der beiden Teststreifen 3 überdeckt.

Vor der Durchführung eines Tests sind keine Linien 341, 342 ersichtlich. Bei der Durchführung eines Tests wird ein Probevolumen einer Probeflüssigkeit durch ein Probeaufgabefenster 42 der Deckschicht 4 auf den Probenaufnahmeabschnitt 32 eines jeden Teststreifens 3 aufgegeben. Durch Kapilarwirkung fließt die Probenflüssigkeit in Richtung Testabschnitt 34. Sie kommt dabei zunächst mit dem Antikörperbelagabschnitt 33 in Kontakt, in dem sich mit Goldpartikeln konjugierte Antikörper zum Antigen eines festzustellenden Analyten in standardisierter (dosierter) Menge befinden. Die Antikörper weisen eine für verschiedene Analyten (Drogen) unterschiedliche Bindungsaktivität auf. Die mit Goldpartikein konjugierten Antikörper werden durch die Probenflüssigkeit mitgenommen, welche den Testabschnitt 34 durchfließt. Enthält die Probenflüssigkeit nicht den festzustellenden Analyten, so werden beim Durchfließen des Testsabschnitts 34 die dort im Bereich der Testlinie 341 befindlichen Antigene des Analyten durch die von der Probenflüssigkeit mitgenommenen Antikörper gebunden und es bildet sich eine sichtbare Testlinie 341 heraus, die durch die mit den Antikörpern konjugierten Goldpartikel eine rote oder braune Farbe erhält. Enthält die Probenflüssigkeit den festzustellenden Analyten, so werden dessen Antigene bereits durch die Antikörper im Bereich des Antikörperbelagabschnitts 33 des Teststreifens 3 gebunden und es kann im Testabschnitt 34 nicht mehr zu einer Anlagerung kommen, weshalb sich keine sichtbare Testlinie 341 herausbildet. Die Kontrolllinie 342 zeigt an, ob der Test valide ist. In Figur 2 ist die Testlinie 341 farbig herausgebildet, weshalb der Test in Bezug des entsprechenden Analyten negativ ist.

Die über die Knickfalz 11 schwenkbar miteinander verbunden Trägerplatten 1 werden derart verschwenkt, dass ihre den Teststreifen 3 gegenüberliegenden Rückseiten aneinanderliegen. Hierdurch ist eine etwa scheckkartengroße Vorrichtung erzielt, die vier Teststreifen 3 aufnimmt. Bei Verwendung üblicher Teststreifen, welche den Test bezüglich dreier Analyten ermöglichen ist so mit der erfindungsgemäßen, im Ausführungsbeispiel scheckkartengroßen Vorrichtung ein Nachweis von zwölf Analyten ermöglicht.

Anstelle der mit Ausnehmungen versehenen Zwischenschicht 2 kann die Trägerplatte 1 auch mit Einprägungen versehen sein, wodurch die Teststreifenaufnahmen 12 und das Reservoir 13 in Form von Vertiefungen in der Trägerplatte 1 ausgebildet sind. Hierdurch kann der Produktionsaufwand weiter minimiert werden.

Die erfindungsgemäße Vorrichtung zeichnet sich dadurch aus, dass sie bei einer kompakten Ausbildung einen Test von einer großen Anzahl von Analyten ermöglicht. Durch die Herstellung der Vorrichtung im Wesentlichen aus Kartonwerkstoffen ist zudem eine umweltgerechte Entsorgung dieser zur einmaligen Verwendung bestimmten Vorrichtung ermöglicht.

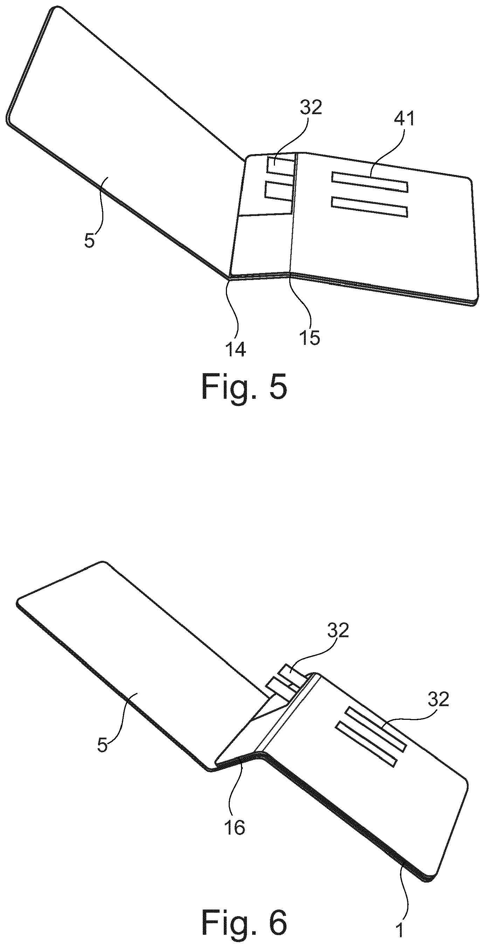

Ausnehmung 43 der Deckschicht 4 in die Trägerplatte 1 mit der auf diesem angeordneten Zwischenschicht 2 eine zweite Knickfalz 15 eingebracht. Über die zweite Knickfalz 15 kann der durch diese begrenzte vordere Abschnitt 16 der Trägerplatte 1 verschwenkt werden, wodurch die Probeaufnahmenabschnitte 32 der beiden Teststreifen 3 freigestellt sind. Zur Durchführung eines Tauchtests kann so der vordere Abschnitt 16 zusammen mit dem Deckel 5 an die Rückseite der Trägerplatte 1 angelegt werden. Unterschiedliche Knickppositionen sind in den Figuren 5 und 6 dargestellt.

Anstelle der mit Ausnehmungen versehenen Zwischenschicht 2 kann die Trägerplatte 1 auch mit Einprägungen versehen sein, wodurch die Teststreifenaufnahmen 12 und das Reservoir 13 in Form von Vertiefungen in der Trägerplatte 1 ausgebildet sind. Hierdurch kann der Produktionsaufwand weiter minimiert werden.

Die erfindungsgemäße Vorrichtung zeichnet sich dadurch aus, dass sie bei einer kompakten Ausbildung einen Test von einer großen Anzahl von Analyten ermöglicht. Durch die Herstellung der Vorrichtung im Wesentlichen aus Kartonwerkstoffen ist zudem eine umweltgerechte Entsorgung dieser zur einmaligen Verwendung bestimmten Vorrichtung ermöglicht. Weiterhin ermöglicht eine gemäß dem zweiten Ausführungsbeispiel ausgebildete Vorrichtung zugleich auch den Einsatz für einen Tauchtest.

## Patentansprüche

1. Vorrichtung zum visuellen Nachweis von Analyten in einer vom menschlichen oder tierischen Organismus stammenden Analysen- oder Flüssigkeitsprobe, umfassend eine Trägerplatte (1) mit wenigstens einer Teststreifenaufnahme (12), in der ein mit einem Probeaufnahmeabschnitt (32) und einem Testabschnitt (34) versehener Teststreifen (3) angeordnet ist, welche wenigstens eine Teststreifenaufnahme (12) zumindest bereichsweise über eine nicht lösbar mit der Trägerplatte (1) verbundenen Deckschicht (4) verschlossen ist, welche Deckschicht (4) im Bereich des Testabschnitts (34) des Teststreifens (3) der wenigstens einen Teststreifenaufnahme (12) ein Kontrollfenster (41) aufweist, **dadurch gekennzeichnet, dass** die Trägerplatte (1) aus Pappe hergestellt ist, wobei vorhandene Teststreifenaufnahmen (12) und/oder Reservoire (13) durch Einprägungen oder durch Ausnehmungen (21) einer zwischen Deckfolie (4) und Trägerplatte (1) angeordneten Zwischenschicht (2) ausgebildet sind.

2. Vorrichtung nach Anspruch 1, **dadurch gekennzeichnet, dass** die Deckschicht (4) ein zu dem Kontrollfenster (41) beabstandet angeordnetes Probeaufgabenfenster (42) aufweist, das eine Probeaufgabe auf den Probeaufnahmeabschnitt (32) wenigstens eines Teststreifens (3) ermöglicht.

3. Vorrichtung nach Anspruch 2, **dadurch gekennzeichnet, dass** wenigstens zwei parallel zueinander angeordnete, jeweils einen Teststreifen (3) aufnehmende Teststreifenaufnahmen (12) angeordnet sind, die an einem Ende in einem gemeinsamen Reservoir (13) münden, in dem die Probeaufnahmeabschnitte (32) der Teststreifen (3) angeordnet sind und über dem das Probeaufgabefenster (42) der Deckschicht (4) positioniert ist.

4. Vorrichtung nach Anspruch 2 oder 3, **dadurch gekennzeichnet, dass** im Bereich des Probeaufgabefensters (42) zwischen Deckschicht (4) und Teststreifen (3) ein Probeaufnahmevlies angeordnet ist.

5. Vorrichtung nach einem der vorgenannten Ansprüche, **dadurch gekennzeichnet, dass** die Deckschicht (4) aus einer Folie gebildet ist, die bevorzugt im Wesentlichen flächig mit der Trägerplatte (1) und den von der Trägerplatte (1) aufgenommenen Teststreifen (3) verklebt ist.

6. Vorrichtung nach einem der vorgenannten Ansprüche, **dadurch gekennzeichnet, dass** das wenigstens eine Kontrollfenster (41) durch jeweils einen transparenten Bereich der Deckschicht (4) gebildet ist.

7. Vorrichtung nach einem der vorgenannten Ansprüche, **dadurch gekennzeichnet, dass** zwei schwenkbar miteinander verbundene Trägerplatten (1) angeordnet sind, die jeweils auf einer Hälfte ihrer Oberfläche mit Teststreifenaufnahmen (12) versehen sind, derart, dass im eingeklappten Zustand jeweils die mit Teststreifenaufnahmen (12) versehene Hälfte einer Trägerplatte (1) an der keine Teststreifenaufnahmen (12) aufweisenden Hälfte der an dieser anliegenden anderen Trägerplatte (1) anliegt.

## Claims

1. Device for visual detection of analytes in an analysis or liquid sample derived from the human or animal organism comprising a support plate (1) with at least one test strip receptacle (12), in which a test strip (3) provided with a sample receiving portion (32) and a test section (34) is arranged, which at least one test strip receptacle (12) is at least partially closed via a cover layer (4) non-detachably connected to the support plate (1), which cover layer (4) has a control window (41) in the area of the test section (34) of the test strip (3) of the at least one test strip receptacle (12), **characterised in that** the support plate (1) is made of cardboard, wherein existing test strip receptacles (12) and/or reservoirs (13) are formed by impressions or recesses (21) of an interlayer (2) arranged between cover film (4) and support plate (1).

2. Device according to claim 1, **characterised in that** the cover layer (4) has a sample application window (42) arranged at a distance from the control window (41), which allows for application of a sample to the sample receiving portion (32) of at least one test strip (3).

3. Device according to claim 2, **characterised in that** at least two test strip receptacles (12) each receiving a test strip (3) are arranged in parallel to one another, which on one end open up into a shared reservoir (13), in which the sample receiving portions (32) of the test strips (3) are arranged and above which the sample application window (42) of the cover layer (4) is positioned.

4. Device according to claim 2 or 3, **characterised in that** in the area of the sample application window (42) between cover layer (4) and test strip (3) a sample application fleece is arranged.

5. Device according to one of the previous claims, **characterised in that** the cover layer (4) is formed by a film, which is preferably substantially laminarly glued to the support plate (1) and the test strips (3) received by the support plate (1).

6. Device according to one of the previous claims, **characterised in that** the at least one control window (41) is formed in each case by one transparent area of the cover layer (4).

7. Device according to one of the previous claims, **characterised in that** two pivotably connected support plates (1) are arranged, which are provided with test strip receptacles (12) on one half of their surface in such a manner that in the folded state, the respective half of one support plate (1) provided with test strip receptacles (12) abuts on the half not provided with any test strip receptacles (12) of the other support plate (1) abutting thereon.

## Revendications

1. Dispositif de détection visuelle d'analytes dans un échantillon à analyser ou un échantillon de liquide provenant de l'organisme humain ou animal, comprenant une plaque support (1) dotée d'au moins un réceptacle (12) pour bandelette réactive dans lequel est disposé une bandelette réactive (3) présentant un segment d'absorption d'échantillon (32) et un segment réactif (34), lequel réceptacle (12) pour bandelette réactive au moins considéré est obturé au moins localement via une couche de couverture (4) reliée de façon non détachable avec la plaque support (1), laquelle couche de couverture (4) présente une fenêtre de contrôle (41) dans la zone du segment réactif (34) de la bandelette réactive (3) d'au moins un réceptacle (12) pour bandelette réactive, **caractérisé en ce que** la plaque support (1) est fabriquée en carton, sachant que les réceptacles (12) pour bandelettes réactives présents et/ou les réservoirs (13) sont formés par des gaufrages ou des évidements (21) d'une couche intermédiaire (2) disposée entre le film de couverture (4) et la plaque support (1).

2. Dispositif selon la revendication 1, **caractérisé en ce que** la couche de couverture (4) présente une fenêtre d'application (42) de l'échantillon disposée à distance de la fenêtre de contrôle (41), laquelle fenêtre première citée permet l'application de l'échantillon sur le segment (32), recevant l'échantillon, d'au moins une bandelette réactive (3).

3. Dispositif selon la revendication 2, **caractérisé en ce que** sont disposés réciproquement parallèles au moins deux réceptacles (12) pour bandelettes réactives recevant chacun une bandelette réactive (3), réceptacles qui à une extrémité aboutissent dans un réservoir commun (13) dans lequel sont disposés les segments (32), recevant l'échantillon, des bandelettes réactives (3) et au-dessus duquel est positionnée la fenêtre (42), d'application de l'échantillon, de la couche de couverture (4).

4. Dispositif selon la revendication 2 ou 3, **caractérisé en ce que** dans la zone de la fenêtre (42) d'application de l'échantillon, entre la couche de couverture (4) et la bandelette réactive (3), est disposé un non-tissé recevant l'échantillon.

5. Dispositif selon l'une des revendications précédentes, **caractérisé en ce que** la couche de couverture (4) est formée par un film qui de préférence est collé à plat contre la plaque support (1) et la bandelette réactive (3) reçue par la plaque support (1).

6. Dispositif selon l'une des revendications précédentes, **caractérisé en ce qu'**au moins une fenêtre de contrôle (41) est formée respectivement par une zone transparente de la couche de couverture (4).

7. Dispositif selon l'une des revendications précédentes, **caractérisé en ce que** deux plaques supports (1) disposées reliées l'une à l'autre de manière pivotante sont munies, respectivement sur une moitié de leur surface, de réceptacles (12) pour bandelettes réactives, de sorte qu'à l'état replié respectivement la moitié d'une plaque support (1) munie de réceptacles (12) pour bandes réactives applique contre la moitié, ne présentant pas de réceptacles (12) pour bandelettes réactives, de l'autre plaque support (1) appliquant contre elle.
